# EUROPEAN PATENT SPECIFICATION

(11) **EP 1 368 041 B1**
(45) Date of publication and mention of the grant of the patent: **01.09.2004**
(21) Application number: 01923963.1
(22) Date of filing: 16.03.2001
(51) Int. Cl.: A61K 31/715, A61K 47/14, A61K 9/127

(54) **AMPHOTERICIN B AQUEOUS COMPOSITION**
WÄSSERIGE ZUSAMMENSETZUNG ENTHALTEND AMPHOTERICIN B
COMPOSITION AQUEUSE D'AMPHOTEROCINE B

(30) Priority: 01.03.2001 IN MU02001217
(43) Date of publication of application: 10.12.2003
(73) Proprietor: Bharat Serums & Vaccines Ltd., Wagle Estate, Thane 400 604 (IN)
(72) Inventor: PAI, Srikanth, Wagle Estate, Thane 400 604 (IN); RIVANKAR, Sangeeta, Wagle Estate, Thane 400 604 (IN)
(74) Representative: Casey, Lindsay Joseph
(86) International application number: PCT/IN2001/000040
(87) International publication number: WO 2002/069983

(56) References cited:
- EP-A- 0 418 153

## Description

### Field of Invention

This invention relates to low toxicity Amphotericin B aqueous composition. This invention is particularly related to the low toxicity Amphotericin B aqueous composition containing phospholipids suitable for parenteral administration.

### Background of the Invention

Amphotericin B is a polyene antifungal, antibiotic drug useful in treatment of invasive fungal infections. However, it has high nephrotoxicity.

The toxicity of the Amphotericin B is reduced by various processes; of these (a) entrapping the drug in liposomes and (b) converting the drug into High drug lipid complex (HDLC) are commonly used.

### Preparation of liposomal Amphotericin B:

In US patent 4973465 (1990) preparation of HDLCs have been described in which sterols like cholesterol are used either alone or in combination with natural phospholipids, phosphatidylcholine.

In US patent 5616334 (1997) a method of preparing liposomal Amphotericin B which involves initially producing blank multilamellar vesicles (MLVs) and then mixing the MLVs with sonicated Amphotericin B suspension in water has been described. This process does not involve the use of any solvents. However, this procedure specifically produces liposomal Amphotericin B which is more toxic than Amphotericin B HDLC. The procedure involves extrusion of blank liposomes for sizing through stacked polycarbonate filters again and again ten times. It also involves removal of unincorporated Amphotericin B by centrifugation after drug loading:

This US patent also describes a process for making HDLC in "Low toxicity drug-lipid systems". In this patent a process for the preparation of Amphotericin B lipid complex has been described. This technique in general is as follows:

**Preparation of HDLCs :** First the drug Amphotericin B is solubilised in a solvent such as Dimethyl sulfoxide (DMSO) or methanol. The lipids, preferably dimyristoylphosphatidylcholine (DMPC) and dimyristoylphosphatidylglycerol (DMPG) in a molar ratio of 7 : 3 are solubilised in solvents such as methanol, ethanol, chlorinated hydrocarbons. The drug solution and the lipid solution are mixed. The solvents are evaporated under reduced pressure, resulting in a thin lipid-drug film. The film is hydrated with an aqueous solution such as water, saline, phosphate buffer saline or glycine buffer, to form HDLCs.

In one variation of the above process, the resulting dry lipid - drug film is resuspended in a solvent, such as methylene chloride and again evaporated under reduced pressure prior to hydrating the film.

In another variation of the above process, the dry lipid-drug film is dehydrated to form flakes; the flakes are then hydrated with aqueous solution.

In another process, the aqueous solution such as saline, buffer or water is added to the solution containing the drug and the lipid, and then the solvent is evaporated off to obtain HDLCs. In this process, formation of thin film of the phospholipids is not required.

In an alternative method for forming the HDLCs described in this US patent, lipid particles (or liposomes) containing bioactive agents, such as Amphotericin B, are formed by first making multilamellar vesicles (MLVs) containing from 6 - 50 mole percent of the bioactive agent. Then subjecting the MLVs to a heating cycle, from about 25°C to about 60°C, most preferably about 60°C. Such a cycle forms a more highly ordered and less toxic Amphotericin B lipid complex.

Another alternate process of making Amphotericin B lipid complex has also been described in this US patent. In that process lipids are admixed with sodium chloride solution (0.9%) and homogenised using a homogeniser. Amphotericin B is dissolved in DMSO and added to the lipid solution while homogenising and homogenised further for about thirty minutes, until the particle size is reduced to about less than 10 microns, preferably to about 10 micron. The resulting lipid particles are size selected following tangential flow filtration. The disadvantage with this process is that the solvent used DMSO has high boiling point and hence difficult to remove from the product. Further trace quantity of DMSO remains in the final product. It is not desirable to have such a solvent in trace quantities in the composition for intravenous administration, as this solvent has been reported to be hepatotoxic (The journal of Infectious diseases 1991 : 164 Pg 418 to 421).

HDLCs are useful preparations to reduce toxicity of Amphotericin B, but the techniques described in US patent 5616334 (1997) require use of large amount of organic solvents, as Amphotericin B has a low solubility in most of the commonly used parenterally acceptable organic solvents. Hence the process involves removal of large quantities of organic solvents by evaporation. Alternatively aprotic solvents such as dimethyl sulfoxide, dimethyl formamide are also used to dissolve Amphotericin B. These aprotic solvents have high boiling point and traces of these solvents is bound to remain in the final composition. As these aprotic solvents are reported to be hepatotoxic, it is not desirable to use these solvents in the process of manufacturing.

There is, therefore, a need to improve the process for large scale manufacture of such Amphotericin B compositions by reducing the quantity of solvents used. That will also bring down the production cost.

The main object of the present invention is to develop a low toxicity parenteral aqueous composition containing Amphotericin B and phospholipids with a view to make it simple and to reduce the cost of manufacture. Further extension of the main object of the present invention is to develop a low toxicity parenteral aqueous composition containing Amphotericin B and phospholipids and not containing traces of DMSO and / or chlorinated hydrocarbons.

### Summary of the Invention :

Accordingly, the present invention relates to a low toxicity parenteral dimethyl sulfoxide free aqueous composition containing Amphotericin B, sodium chloride and phospholipids.

The present invention further relates to a process for manufacture of a low toxicity parenteral dimethyl sulfoxide free aqueous composition containing Amphotericin B, sodium chloride and phospholipids comprising steps of
(i) dissolving one or more phospholipids in one or more of organic solvents selected from a group of parenterally acceptable solvents such as methanol, ethanol, isopropyl alcohol, chloroform, carbon tetrachloride and methylene chloride and then removing the solvents by evaporation under reduced pressure to form a dry film of the single or mixed phospholipids;
(ii) suspending Amphotericin B in a parenterally acceptable aqueous phase, not containing sodium chloride or suspending micronised Amphotericin B in a parenterally acceptable aqueous phase, which may contain sodium chloride;
(iii) adding aqueous phase containing suspended Amphotericin B formed at the end of step (ii) to said film of phospholipids obtained at the end of step (i) and mixing the two to obtain a suspension of said Amphotericin B together with said phospholipids in said aqueous phase;
(iv) adjusting the pH of said suspension obtained at the end of step (iii) to 6.0 - 8.0 and then homogenising it till it becomes filterable through a 2µ glass fibre filter;
(v) adding sufficient sodium chloride solution in water at the end of step (iv) so that the sodium chloride content of the final product is at least 0.1% w/v;
(vi) filtering said homogenised suspension obtained at the end of step (v) through a 2µ glass fibre filter and filling the filtrate in vials under nitrogen cover, sealing the vials and sterilising the sealed vials by autoclaving to obtain the final product suitable for parenteral administration.

The present invention also relates to a low toxicity parenteral Amphotericin B aqueous composition, containing atleast 0.1% w/v sodium chloride and phospholipids as described herein and made by the process of the present invention as described above.

### Detailed description of embodiments of the invention

The content of Amphotericin B in the composition of present invention varies from 0.1% w/v to 1.0% w/v of the composition, preferably the content of Amphotericin B is 0.5%w/v of the composition.

The total content of phospholipids varies from 0.1% w/v to 1.0%w/v of the composition. The preferred content is from about 0.4% to about 0.6%w/v.

The weight ratio of Amphotericin B to phospholipids is from about 1:0.5 to about 1:1.5. The preferred weight ratio is from about 1:0.8 to about 1: 1.2.

In this process, phospholipids are chosen from egg phosphatidylcholine, or a mixture of dimyristoylphosphatidylcholine (DMPC) and dimyristoylphosphatidylglycerol sodium salt (DMPG). When a mixture of two phospholipids DMPC and DMPG are used, then the weight ratio of phospholipids DMPC : DMPG is between 7:1 and 7:15, preferably 7:3.

The solvents used for dissolving the phospholipids are chosen from alcoholic solvents such as ethanol, methanol, Isopropyl alcohol with or without addition of chlorinated hydrocarbons such as chloroform, methylene chloride, carbon tetrachloride. Alcoholic solvents alone or chlorinated hydrocarbons alone can be used for dissolving the phospholipids. Alternatively alcoholic solvents and chlorinated hydrocarbons can also be used in combination to dissolve the phospholipids. When chlorinated hydrocarbons are not selected, the composition is free from chlorinated hydrocarbons. Preferred solvent used for dissolving phospholipids is ethanol.

Micronised Amphotericin B wherever used in this invention is micronised using air jet mill to particle size less than 10 microns.

The pH of aqueous phase used for dispersing Amphotericin B is adjusted to 6.0 - 8.0 using dilute sodium hydroxide solution whenever buffer solution is not used in the composition.

The aqueous phase used for suspending Amphotericin B is parenterally acceptable vehicle such as water or phosphate buffer. When micronised Amphotericin B is used, the aqueous phase used for suspending Amphotericin B can be saline, phosphate buffer saline, water or phosphate buffer.

Sodium chloride is added as a solution in water after homogenisation and before filtration. However sodium chloride can be added at any step (ii) to (iv) of manufacturing specified under "Summary of the invention" when micronised Amphotericin B is used.

The concentration of Sodium chloride is from about 0.1% to 0.9% w/v of the composition preferably 0.4% to 0.9% w/v of the composition.

Homogenisation is carried out using high pressure homogeniser at not less than 5000 psi till the product is filterable through 2 micron glass fibre filter.

In another embodiment of the invention, the Amphotericin B lipid suspension is sonicated in a bath sonicator before homogenisation to get the uniform suspension after adjusting the pH to about 6.0 - 8.0. Dilute sodium hydroxide solution is used to adjust the pH whenever buffer solution is not used in the composition.

The homogenised Amphotericin B lipid suspension is filtered through 2µ glass fibre filters following the usual filtration procedure under pressure either using filtered Nitrogen or filtered compressed air.

After filtration, the homogenised suspension is filled into vials under nitrogen cover and sterilised by conventional autoclaving at 110°C to 121°C, preferably at 121°C for 20 minutes or 110°C for 40 minutes. The sterilisation can also be carried out by specialised process of autoclaving in which the heating and cooling cycle time is reduced by rapid heating and rapid cooling system.

In the earlier process of preparing HDLCs as described in US patents 4973465 (1990) and 5616334 (1997), Amphotericin B is dissolved in very large amount of organic solvents. In one of the examples in US patent 5616334 (1997), for preparing 1 vial of 20 ml of Amphotericin B lipid complex equivalent to 100mg of Amphotericin B, one litre of methanol is used. In another example to decrease the solvent volume, 5 ml of DMSO for 100mg of Amphotericin B has been used, but DMSO is not a recommended solvent for intravenous injection as DMSO has been reported to be hepatotoxic.

In the process of the present invention, Amphotericin B is not at all dissolved in any solvents while the conventional process use DMSO for dissolving Amphotericin B.

In the process of present invention, when Amphotericin B, suspended in an aqueous phase containing sodium chloride, was added to the lipid film and homogenised, it was observed to form aggregates and the homogenised product was not filterable through 2 micron glass fibre filter by the usual filtration procedure.

After extensive experimentation, we found that when aqueous phase used for suspending Amphotericin B was prepared without addition of any sodium chloride in it, homogenisation proceeded smoothly without any aggregation of the suspension and the homogenised bulk was filterable.

However, during the course of this invention, we found that sodium chloride is essential in the composition to reduce toxicity. Amphotericin B aqueous compositions containing different concentrations of sodium chloride at a dose of 80 mg/kg body weight, were injected in mice, in a group of eight. Amphotericin B aqueous composition without any sodium chloride as described in the Examples below were prepared and injected separately. Before each injection, volume equivalent to a dose of 80 mg/kg body weight was diluted to 0.5 ml with 5% dextrose injection to render it isotonic. The percentage mortality observed at the end of 72 hours are as shown in Table 1.

**Table 1**

| **Concentration of Sodium chloride in Amphotericin B aqueous composition** | **Prepared as per Example** | **Percentage mortality in mice at 80mg/kg dose** |
|---|---|---|
| 0.9% w/v | III | Nil |
| 0.7% w/v | IV | Nil |
| 0.4% w/v | V | Nil |
| 0.1% w/v | VI | 50% |
| Nil | XIII | 87.5% |

From the Table 1, it is clear that a minimum concentration of 0.1% sodium chloride is essential to reduce toxicity. Hence during the hydration of phospholipid film, aqueous phase without any sodium chloride was used to make homogenisation smooth and filtration through 2µ glass fibre filter easy. Sodium chloride was added as a solution after the process of homogenisation. Addition of Sodium chloride in the composition of the present invention is essential to reduce toxicity of Amphotericin B. Eventhough with the addition of 0.1% w/v sodium chloride, the LD₅₀ was 80mg/kg, this LD₅₀ is very much higher than the conventional Amphotericin B preparations containing sodium desoxycholate which is reported to be around 4mg/kg.

After a lot of further experiments, we found that reducing the average particle size of Amphotericin B to less than 10 microns by micronising, helped in overcoming the problem of aggregation during homogenisation. The particle size analysis of Amphotericin B before and after micronisation was carried out with Sympatic HELOS Particle Size Analyser. Micronisation of Amphotericin B also helped in overcoming the problem of filtration associated with the presence of sodium chloride in the homogenised aqueous suspension containing non-micronised Amphotericin B and phospholipid.

Filtration of homogenised bulk prepared using micronised Amphotericin B is easy and commonly used filters can be used; in the prior art process such as in US patent 5616334 (1997), filtering of HDLC is performed through a tortorous path or straight through a membrane filter such as a polycarbonate filter.

Thus in one embodiment of the invention, we could overcome the problem of aggregation and filtration using non-micronised Amphotericin B by avoiding addition of sodium chloride to the aqueous phase. However, we found that addition of sodium chloride in atleast a minimum concentration of 0.1% w/v is essential to reduce the toxicity of Amphotericin B aqueous composition. Having sodium chloride in it, we solved the problem of aggregation and filtration by deferring the addition of Sodium chloride until the step of homogenisation.

Accordingly, in the first embodiment of the invention, in the process for manufacture of a low toxicity sterile Amphotericin B aqueous composition containing atleast 0.1% w/v sodium chloride, the aqueous phase used for dispersing non-micronised Amphotericin B is water or phosphate buffer not containing sodium chloride. Sodium chloride is added just after homogenisation of the aqueous suspension containing non-micronised Amphotericin B and the phospholipids.

In the second embodiment of the invention, the problem of aggregation and filtration of the suspension of the phospholipid and the aqueous phase containing Amphotericin B, was solved by using micronised Amphotericin B.

In combination of the two embodiments, when the Amphotericin B used is micronised and is suspended in an aqueous phase not containing sodium chloride, sodium chloride is added into the aqueous phase before, during or after homogenisation step.

### Examples :

The invention will now be illustrated by way of Examples. The Examples are by way of illustration only and in no way restrict the scope of the invention.

There are 4 groups of Examples as described below in the Table 2.

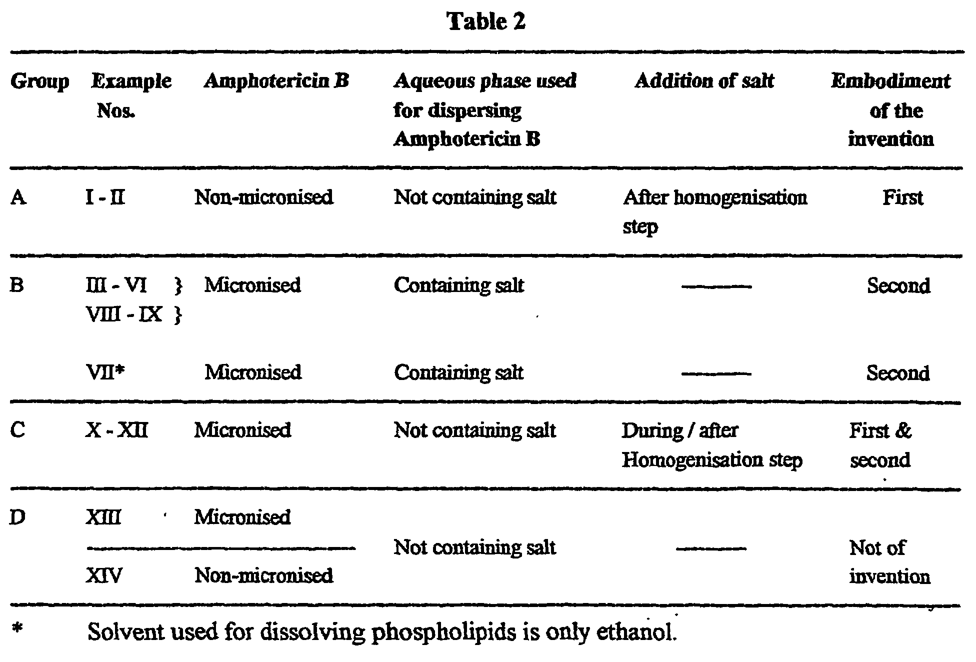

All the raw materials used in these Examples were of parenteral grade. Equipments used were of conventional nature. Entire processing was done in an area with a controlled environment required for manufacturing sterile products.

Amphotericin B used in these Examples was of parenteral grade obtained from Alpharma complying with USP specifications. Micronised Amphotericin B wherever used in these Examples was prepared by micronising Amphotericin B using air jet mill to the particle size of less than 10 microns.

Phospholipids DMPC and DMPG used in the Examples were of parenteral grade and were procured from Avanti Polar Lipids.

Phospholipid Egg phosphatidylcholine used in the Examples was of parenteral grade and was procured from Lipoids

Organic solvents used in the Examples were of AR (Analytical reagent) quality.

Phosphate buffer used in the Examples were prepared as per Indian Pharmacopoeia.

Phosphate buffer saline pH 7.4 used in the Example was prepared by dissolving 1.19gm of Disodium hydrogen orthophosphate, 0.095gms of Potassium dihydrogen orthophosphate and 4gms of Sodium chloride in 400ml of water. Water was added to make up the volume to 500ml.

### GROUP A : Example I & II

The ingredients used in these Examples are shown in Table 3 :

**Table 3**

| | | **Example I** | **Example II** |
|---|---|---|---|
| a) | Amphotericin B | 1.00g | 1.00g |
| b) | DMPC | 0.68g | 0.68g |
| c) | DMPG | 0.30g | 0.30g |
| d) | Ethanol* | 200ml | 200ml |
| e) | Chloroform* | 10 ml | 10ml |
| f) | pH - at dispersion | 6.95** | 7.2 |
| | before homogenisation | 6.80** | 7.2 |
| g) | Sodium chloride | 1.80g | 1.80g |
| h) | Water q. s.to | 200ml | -- |
| i) | Phosphate buffer pH 7.2 q.s.to | -- | 200ml |

| | | | |
|---|---|---|---|
| * Does not remain in the final product. | | | |
| ** Adjusted using 0.1N Sodium hydroxide solution | | | |

### Procedure:

In Example I, Amphotericin B was suspended in 150ml of water under stirring and under nitrogen bubbling. The pH was adjusted to about 6.95 with 0.1 N Sodium hydroxide solution.

In Example II, Amphotericin B was suspended in 150ml of Phosphate buffer pH 7.2 under stirring and under nitrogen bubbling.

Phospholipids DMPC and DMPG were dissolved in Chloroform in a rotary flask. Ethanol was added after complete dissolution of phospholipids and allowed to mix by rotating the flask at moderate speed under nitrogen flushing. This alcoholic solution was rotary evaporated under reduced pressure to complete dryness. Nitrogen was flushed for 30 min. after complete removal of solvents.

The dry lipid film was hydrated in the rotary flask with aqueous suspension of Amphotericin B prepared as above keeping the flask under continuous rotation with continuous flushing of nitrogen. pH of Amphotericin B lipid suspension obtained in Example I was adjusted to about 6.80 with 0.1N Sodium hydroxide solution. The content of the flask was sonicated in a bath sonicator for 1 hr. The volume was made upto 180 ml with water in Example I and with phosphate buffer pH 7.2 in Example II.

The Amphotericin B Lipid suspension was then homogenised using APV high pressure homogeniser till the homogenised product was filterable through 2µ glass fibre filter.

Sodium Chloride was dissolved in water and diluted to 20ml with water in Example I. In Example II, sodium chloride was dissolved in and diluted to 20ml with phosphate buffer pH 7.2. This sodium chloride solution was added to the homogenised Amphotericin B Lipid suspension under low speed stirring and nitrogen flushing. This product was transferred back to the homogeniser and recirculated for 5 minutes without applying pressure. Then it was filtered through a 2µ glass fibre filter and filled into glass containers under nitrogen, sealed and autoclaved at 110°C for 40 mins. In Example II autoclaving is done at 110°C for 40 minutes with rapid heat and rapid cooling cycle.

### GROUP B :

### 1) Example III to VI

The ingredients used in these Examples are shown in Table 4 with the procedure given below, quantity of sodium chloride added has been changed from 1.8g to 0.2g.

**Table 4**

| | | **Examples** | | | |
|---|---|---|---|---|---|
| | | **III** | **IV** | **V** | **VI** |
| a) | Amphotericin B | 1g | 1g | 1g | 1g |
| | (micronised) | | | | |
| b) | DMPC | 0.68g | 0.68g | 0.68g | 0.68g |
| c) | DMPG | 0.30g | 0.30g | 0.30g | 0.30g |
| d) | Sodium Chloride | 1.80g | 1.40g | 0.80g | 0.20g |
| e) | Ethanol* | 200ml | 200ml | 200ml | 200ml |
| f) | Chloroform* | 10ml | 10ml | 10ml | 10ml |
| g) | pH - at dispersion | 7.20** | 7.15** | 7.05** | 7.20** |
| | before homogenisation | 7.00** | 7.10** | 7.15** | 7.00** |
| h) | Water q.s.to | 200ml | 200ml | 200ml | 200ml |

| | | | | | |
|---|---|---|---|---|---|
| * Does not remain in the final product. | | | | | |
| ** Adjusted using 0.1N Sodium hydroxide solution | | | | | |

### Procedure:

Phospholipids DMPC and DMPG were dissolved in Chloroform in a rotary flask. Ethanol was added after complete dissolution of phospholipids and allowed to mix by rotating the flask at moderate speed under nitrogen flushing. This alcoholic solution was rotary evaporated under reduced pressure to complete dryness. Nitrogen was flushed for 30 min. after complete removal of solvents.

Sodium chloride was dissolved in 175ml of water. Nitrogen was bubbled in this solution for 15 min. Micronised Amphotericin B was then suspended in the sodium chloride solution under stirring and under nitrogen bubbling. The pH was adjusted by addition of 0.1N sodium hydroxide to the values as shown in Table 4 for each Example.

The dry lipid film was hydrated in the rotary flask with aqueous suspension of Amphotericin B prepared as above, keeping the flask under continuous rotation with continuous flushing of nitrogen. pH of this Amphotericin B lipid complex obtained was adjusted as shown Table 4. The content of the flask was sonicated in a bath sonicator for 1 hr.

The volume was made upto 200 ml with water.

The Amphotericin B Lipid suspension was then homogenised using high pressure homogeniser till the product was filterable through 2µ glass fibre filter.

The homogenised Amphotericin B Lipid suspension was filtered through a 2µ glass fibre filter and filled into glass containers under nitrogen, sealed and autoclaved at 110°C for 40 mins with rapid heat and rapid cooling cycle.

Toxicity studies in mice with the product of Examples III, IV and V at a dose of 80mg/kg body weight did not show any mortality while that of Example VI showed 50% mortality.

### Group B continued.....

### 2) Example VII to IX

The ingredients used in these Examples are shown in Table 5 with the procedure given below

**Table 5**

| | | **Examples** | | |
|---|---|---|---|---|
| | | **VII** | **VIII** | **IX** |
| a) | Amphotericin B (micronised) | 1g | 1g | 1g |
| b) | DMPC | 0.68g | -- | 0.68g |
| c) | DMPG | 0.30g | -- | 0.30g |
| d) | Egg phosphatidylcholine | -- | 0.90g | -- |
| e) | Sodium Chloride | 1.80g | 1.80g | *** |
| f) | Ethanol* | 300ml | 200ml | 200ml |
| g) | Chloroform* | - | 15ml | 10ml |
| h) | pH - at dispersion | 7.15** | 7.15** | 7.40 |
| | before homogenisation | 7.05** | 6.95** | 7.40 |
| i) | Water q. s.to | 200ml | 200ml | -- |
| j) | Phosphate buffer saline q.s.to | - | -- | 200ml |

| | | | | |
|---|---|---|---|---|
| * Does not remain in the final product. | | | | |
| ** Adjusted using 0.1N sodium hydroxide solution. | | | | |
| *** ≈ 2gm contributed from PBS. | | | | |

### Procedure :

In the Example VII, phospholipids DMPC and DMPG were dissolved in ethanol in a rotary flask by rotating the flask at moderate speed under nitrogen flushing.

In the Example VIII, phospholipid Egg phosphatidylcholine was dissolved in Chloroform in a rotary flask and in the Example IX phospholipids DMPC & DMPG were dissolved in chloroform in a rotary flask. Ethanol was added after complete dissolution of phospholipids in chloroform and allowed to mix by rotating the flask at moderate speed under nitrogen flushing.

In these Examples the phospholipid solutions thus obtained were rotary evaporated under reduced pressure to complete dryness. Nitrogen was flushed for 30 min. after complete removal of the solvent.

In Example VII & VIII, sodium chloride was dissolved in 175ml of water. Nitrogen was bubbled in this solution for 15 min. Micronised Amphotericin B was then suspended in the sodium chloride solution under stirring and under nitrogen bubbling, the pH was adjusted to about 7.15 with 0.1 N Sodium hydroxide solution.

In Example IX, micronised Amphotericin B was suspended in 175ml of phosphate buffer saline pH 7.4 (PBS) under stirring. Nitrogen was bubbled for 15 minutes. PBS contributes about 2gms of sodium chloride.

The dry lipid film was hydrated in the rotary flask with aqueous suspension of micronised Amphotericin B prepared as above keeping the flask under continuous rotation with continuous flushing of nitrogen. pH of this Amphotericin B lipid suspension obtained was adjusted to 7.05 in Example VII and to 6.95 in Example VIII with 0.1N Sodium hydroxide solution.

The volume was made upto 200 ml with water in Example VII & VIII.

The volume was made upto 200 ml with phosphate buffer saline pH 7.4 in Example IX.

The Amphotericin B Lipid suspension was then homogenised using APV high pressure homogeniser till the homogenised product was filterable through 2µ glass fibre filter.

The homogenised Amphotericin B Lipid suspension was filtered through a 2µ glass fibre filter and filled into glass containers under nitrogen, sealed and autoclaved at 121°C for 20 minutes in Example IX and at 110°C for 40 minutes in Example VII & VIII.

The Sterile Amphotericin B aqueous composition obtained in Example VII was subjected to toxicity studies in mice and stability studies. The results of the toxicity study are given in Table 6 and stability study are given in Table 7.

### Particle size arralysis :

Particle size of the Amphotericin B aqueous composition obtained in Example VII was evaluated on Model 770 AccuSizer of Particle Sizing Systems, Inc., USA. 95% of the particles were found to be below 1.63µ in size and 90% of the particles were found to be below 1.28µ in size.

### Toxicity study in mice :

The toxicity study of the Amphotericin B aqueous composition obtained in Example VII was studied in mice along with a conventional Amphotericin B product containing sodium desoxycholate. The followings are the observations:

**Table 6**

| **Acute toxicity study in mice** | |
|---|---|
| LD₅₀ (Intravenous) Amphotericin B conventional product - | 3.5mg/kg body weight |
| Amphotericin B aqueous composition of Example VII - | >80mg/kg body weight |

The LD₅₀ of Amphotericin B aqueous composition prepared in this laboratory after single injection was >80 mg/kg in mice. This was more than 20 times higher than LD₅₀ after a single injection of conventional Amphotericin B product containing sodium desoxycholate.

**Table 7**

| **Stability data for Amphotericin B aqueous composition** **of Example VII at recommended storage temperature of 2°C - 8°C** | | |
|---|---|---|
| **PERIOD** | **APPEARANCE** | **AMPHOTERICIN B CONTENT** |
| Initial | Yellow coloured suspension which settles on keeping and disperses uniformly on mild shaking | 100.6% |
| 6 Months | Yellow coloured suspension which settles on keeping and disperses uniformly on mild shaking | 100.3% |
| 1 Year | Yellow coloured suspension which settles on keeping and disperses uniformly on mild shaking | 99.8% |
| 18 Months | Yellow coloured suspension which settles on keeping and disperses uniformly on mild shaking | 98.3% |
| 2 Years | Yellow coloured suspension which settles on keeping and disperses uniformly on mild shaking | 96.5% |

This example clearly shows that parenteral Amphotericin B aqueous composition having not even a trace of DMSO or chlorinated hydrocarbon when prepared by the improved process of the present invention where these solvents are not at all used, complies with general requirements of a marketable injectable suspension product. The novel aqueous composition prepared by the process of Example VII is totally free from harmful solvents such as DMSO and chlorinated hydrocarbons.

### GROUP C : Example X to XII

The ingredients used in these Examples are shown in Table 8 with the procedure given below

**Table 8**

| | | **Examples** | | |
|---|---|---|---|---|
| | | **X** | **XI** | **XII** |
| a) | Amphotericin B | | | |
| | (micronised) | 1g | 1g | 1g |
| b) | DMPC | 0.68g | 0.68g | 0.68g |
| c) | DMPG | 0.30g | 0.30g | 0.30g |
| d) | Sodium Chloride | 1.80g | 1.80g | 1.80g |
| e) | Ethanol* | 200ml | 200ml | 200ml |
| f) | Chloroform* | 10ml | 10ml | 10ml |
| g) | pH - at dispersion | 7.15** | 7.30** | 7.2 |
| | before homogenisation | 6.90** | 7.00** | 7.2 |
| h) | Water q.s.to | 200ml | 200ml | -- |
| i) | Phosphate buffer pH 7.2 q.s. to | - | -- | 200ml |

| | | | | |
|---|---|---|---|---|
| * Does not remain in the final product | | | | |
| ** Adjusted using 0.1N Sodium hydroxide solution. | | | | |

### Procedure:

Phospholipids DMPC and DMPG were dissolved in Chloroform in a rotary flask Ethanol was added after complete dissolution of phospholipids and allowed to mix by rotating the flask at moderate speed under nitrogen flushing. This alcoholic solution was rotary evaporated under reduced pressure to complete dryness. Nitrogen was flushed for 30 min. after complete removal of solvents.

Micronised Amphotericin B was suspended in 150ml of water in Example X and XI and in 150ml of phosphate buffer pH 7.2 in Example XII under stirring and under nitrogen bubbling. The pH was adjusted by addition of 0.1N sodium hydroxide to the values as shown in Table 8 for examples X & XI.

The dry lipid film was hydrated in the rotary flask with micronised Amphotericin B suspension prepared as above using the rotary evaporator with continuous flushing of nitrogen. pH of Amphotericin B lipid suspension obtained was adjusted with 0.1N Sodium hydroxide solution as shown in Table 8. In Examples X and XI, the volume was made upto 180ml with water while in Example XII, the volume was made upto 180ml with Phosphate buffer pH 7.2. In Example XI, the content of the flask was sonicated in a bath sonicator for 1 hour.

In Example X & XII, the Amphotericin B Lipid suspension was then homogenised using APV high pressure homogeniser till the homogenised product was filterable through 2µ glass fibre filter.

Sodium chloride was dissolved in water and diluted to 20ml with water in Example X. Sodium chloride was dissolved in phosphate buffer of pH 7.2 and diluted to 20ml with phosphate buffer of pH 7.2 in Example XII. This sodium chloride solution was added to the homogenised Lipid suspension under low speed stirring and nitrogen flushing. This product was transferred back to the homogeniser and recirculated for 5 minutes without applying pressure.

In Example XI, the Amphotericin B lipid suspension after sonication was passed through homogeniser 3 times under pressure using APV high pressure homogeniser. Sodium chloride was dissolved in water and diluted to 20ml with water. This sodium chloride solution was added under low speed mixing to the Amphotericin B lipid suspension obtained at the end of 3 passes. This product was transferred back to the homogeniser and recirculated for 5 minutes without applying pressure. This was again homogenised under pressure till the product was filterable through 2µ glass fibre filter.

The product was then filtered through a 2µ glass fibre filter. The filtered product was filled into glass containers under nitrogen, sealed and autoclaved at 110°C for 40 minutes with rapid heat and rapid cooling cycle.

### GROUP D: Example XIII and XIV

The ingredients used in these Examples are shown in Table 9 with the procedure given below

**Table 9**

| | | **Example XIII** | **Example XIV** |
|---|---|---|---|
| a) | Amphotericin B (micronised) | 1g | -- |
| b) | Amphotericin B (non-micronised) | -- | 1g |
| c) | DMPC | 0.68g | 0.68g |
| d) | DMPG | 0.30g | 0.30g |
| e) | Ethanol* | 200ml | 200ml |
| f) | Chloroform* | 10 ml | 10ml |
| g) | Water q.s.to | 200ml | 200ml |
| h) | pH - at dispersion | 7.25** | 7.20** |
| | before homogenisation | 7.15** | 7.10** |

| | | | |
|---|---|---|---|
| * Does not remain in the final product | | | |
| ** Adjusted using 0.1N Sodium hydroxide solution | | | |

### Procedure :

Phospholipids DMPC and DMPG were dissolved in Chloroform in a rotary flask. Ethanol was added after complete dissolution of phospholipids and allowed to mix by rotating the flask at moderate speed under nitrogen flushing. This alcoholic solution was rotary evaporated under reduced pressure to complete dryness. Nitrogen was flushed for 30 min. after complete removal of solvents.

In Example XIII, micronised Amphotericin B was suspended in water under stirring and under nitrogen bubbling. In Example XIV, non-micronised Amphotericin B was suspended in water under stirring and under nitrogen bubbling. The pH was adjusted with 0.1 N Sodium hydroxide solution as shown in Table 9.

The dry lipid film was hydrated in the rotary flask with Amphotericin B suspension prepared as above using the rotary evaporator with continuous flushing of nitrogen. pH was adjusted with 0.1N Sodium hydroxide solution as shown Table 9. In Example XIV, the content of the flask was sonicated for 1 hr. The volume was made upto 200 ml with water.

The Amphotericin B Lipid suspension was then homogenised using APV high pressure homogeniser till the homogenised product was filterable through 2µ glass fibre filter.

The homogenised Amphotericin B Lipid suspension was filtered through a 2µ glass fibre filter. The filtered product was filled into glass containers under nitrogen, sealed and autoclaved. Autoclaving was done at 121°C for 20 minutes.

### Toxicity study in mice :

The toxicity of Amphotericin B aqueous composition (without sodium chloride) obtained in Example XIII and XIV was studied in mice along with Amphotericin B aqueous composition containing sodium chloride as per Example III. The followings are the observations:

**Table 10**

| **Acute toxicity study in mice** | |
|---|---|
| LD₅₀ (Intravenous) Amphotericin B aqueous composition (with sodium chloride) as per Example III - | > 80mg/kg body weight |
| Amphotericin B aqueous composition (without sodium chloride) as per Example XIII & XIV - | 40mg/kg body weight |

The LD₅₀ of Amphotericin B aqueous composition prepared without sodium chloride as per Example XIII and XIV after single injection was 40 mg/kg body weight in mice as compared to >80 mg/kg with Amphotericin B aqueous composition prepared with sodium chloride as per Example III. This proves that sodium chloride is required to form Amphotericin B aqueous composition of low toxicity.

### ADVANTAGES OF THE INVENTION:

The advantages of the present invention are given below:
i) In present invention, Amphotericin B aqueous composition has been prepared without the use of DMSO which has been reported to be hepatotoxic.
ii) Due to low solubility of Amphotericin B in parenterally acceptable organic solvents, (0.1 mg/ml in methanol) a large volume of organic solvent is required which makes the process more tedious, time consuming and commercially not feasible. The process of the present invention does not require, dissolving Amphotericin B in any organic solvent
iii) The process of the prior art requires a specialised technique for filtration like tangential flow filtration or extrusion. In the process of present invention, conventional filtration is used.
iv) The product prepared by the process of present invention, is stable to sterilisation by autoclaving thus making it suitable for intravenous use.

Process of present invention is simple and cost effective, and an improvement over the process of prior art. The most important feature of the process of this invention is the greatest purity of the obtained product without the risk of retaining any traces of harmful solvents because such solvents are not at all used in the process.

## Claims

1. A process for manufacture of a low toxicity parenteral dimethyl sulfoxide free aqueous composition containing Amphotericin B, sodium chloride and phospholipids, comprising steps of
(i) dissolving one or more phospholipids in one or more, of parenterally acceptable organic solvents and then removing the solvents by evaporation under reduced pressure to form a dry film of the single or mixed phospholipids;
(ii) suspending Amphotericin B in a parenterally acceptable aqueous phase, not containing sodium chloride or suspending micronised Amphotericin B in a parenterally acceptable aqueous phase, which may contain sodium chloride;
(iii) adding aqueous phase containing suspended Amphotericin B formed at the end of step (ii) to said film of phospholipids obtained at the end of step (i) and mixing the two to obtain a suspension of said Amphotericin B together with said phospholipids in said aqueous phase;
(iv) adjusting the pH of said suspension obtained at the end of step (iii) to 6.0 - 8.0 and then homogenising it till it becomes filterable through a 2µ glass fibre filter; and
(v) adding sufficient sodium chloride solution in water at the end of step (iv) so that the sodium chloride content of the final product is at least 0.1% w/v.

2. A process as claimed in Claim 1, further comprising:
(vi) filtering said homogenised suspension obtained at the end of step (v) through a 2µ glass fibre filter and filling the filtrate in vials under nitrogen cover, sealing the vials and sterilising the sealed vials by autoclaving to obtain the final product suitable for parenteral administration.

3. A process as claimed in Claim 1 or Claim 2, wherein the phospholipids are chosen from egg phosphatidylcholine (EPC) or a mixture of dimyristoyl phosphatidylcholine (DMPC) and dimyristoylphosphatidylglycerol sodium salt (DMPG).

4. A process as claimed in any one of the preceding claims, wherein said organic solvent is selected from alcoholic solvents, chlorinated hydrocarbons and mixtures thereof.

5. A process as claimed in Claim 4, wherein said organic solvent is selected from methanol, ethanol, isopropyl alcohol, chloroform, carbon tetrachloride and methylene chloride.

6. A process as claimed in Claim 4, wherein said organic solvent is ethanol.

7. A process as claimed in any one of the preceding claims, wherein the content of Amphotericin B is from about 0.1% to 1% w/v of the composition

8. A process as claimed in Claim 7, wherein the content of Amphotericin B is 0.5% w/v of the composition.

9. A process as claimed in any one of the preceding claims, wherein the content of sodium chloride is between 0.1% to 0.9% w/v of the composition:

10. A process as claimed in Claim 9, wherein the content of sodium chloride is between 0.4% to 0.9% w/v of the composition.

11. A process as claimed in any one of the preceding claims, wherein the content of phospholipids is from about 0.1% to 1% w/v of the composition.

12. A process as claimed in any one of the preceding claims, wherein the content of phospholipids is 0.4% to 0.6% w/v of the composition.

13. A process as claimed in any one of the preceding claims, wherein the weight ratio of Amphotericm B to phospholipids is from about 1 : 0.8 to about 1:1.2

14. A process as claimed in any one of the preceding claims, wherein phospholipids are dimyristoylphosphatidylcholine (DMPC) and dimyristoylphosphatidylglycerol (DMPG) in a DMPC:DMPG weight ratio from about 7:1 to about 7:15.

15. A process as claimed in Claim 14 wherein the DMPC:DMPG weight ratio is 7:3.

16. A process as claimed in any one of the preceding claims, wherein non-micronised Amphotericin B is used and the parenterally acceptable aqueous phase used in step (ii) of Claim 1 is water or phosphate buffer.

17. A process as claimed in any one of Claims 1 to 15, wherein micronised Amphotericin B is used and the parenterally acceptable aqueous phase used in step (ii) of Claim 1 is water, phosphate buffer, saline or phosphate buffer saline.

18. A process as claimed in any one of the preceding claims, wherein the pH of said aqueous phase used for suspension of Amphotericin B at step (ii) is adjusted to 6.0 - 8.0

19. A process as claimed in any one of the preceding claims, wherein sterilisation of the homogenised filtered suspension is carried out by conventional autoclaving.

20. A process as claimed in any one of the preceding claims, wherein the sterilisation temperature is 110°C.

21. A process as claimed in any one of Claims 1 to 18 and 20, wherein sterilisation is carried out by a specialised process of autoclaving in which the heating and cooling time is reduced by rapid heat and rapid cool cycle.

22. A process as claimed in any one or Claims 1 to 15 and 17 to 21, wherein the Amphotericin B is micronised and sodium chloride is added at any of steps (ii) to (iv) so that the sodium chloride content of the final product is at least 0.1% w/v.

23. A process as claimed in any one of the preceding claims, wherein the aqueous composition product is totally free from any chlorinated hydrocarbon.

24. A low toxicity parenteral dimethyl sulfoxide free aqueous composition containing Amphotericin B, sodium chloride and phospholipids obtainable by a process as claimed in any one of the preceding claims.

## Patentansprüche

1. Verfahren zur Herstellung einer parenteralen, Dimethylsulfoxid-freien wässrigen Zusammensetzung von geringer Toxizität, die Amphotericin B, Natriumchlorid und Phospholipide enthält, umfassend die Schritte von:
(i) Auflösen von einem oder mehr Phospholipid(en) in einem oder mehr parenteral verträglichen organischen Lösungsmittel(n) und dann Entfernen der Lösungsmittel durch Verdampfung unter reduziertem Druck zur Bildung eines Trockenfilms aus den einzelnen oder gemischten Phospholipiden;
(ii) Suspendieren von Amphotericin B in einer parenteral verträglichen wässrigen Phase, die kein Natriumchlorid enthält, oder Suspendieren von mikronisiertem Amphotericin B in einer parenteral verträglichen wässrigen Phase, die Natriumchlorid enthalten kann;
(iii) Zufügen der wässrigen Phase, enthaltend am Ende von Schritt (ii) gebildetes suspendiertes Amphotericin B zu genanntem Film aus Phospholipiden, der am Ende von Schritt (i) erhalten wurde und Mischen der beiden zum Erhalt einer Suspension von genanntem Amphotericin B zusammen mit genannten Phospholipiden in genannter wässriger Phase;
(iv) Einstellen des pH der am Ende von Schritt (iii) erhaltenen genannten Suspension auf 6,0 - 8,0 und dann ihr Homogenisieren, bis sie durch ein Glasfaserfilter von 2 µ filtrierbar ist; und
(v) Zufügen von ausreichender Natriumchloridlösung in Wasser am Ende von Schritt (iv), damit der Natriumchloridgehalt des Endproduktes mindestens 0,1 % (w/v) beträgt.

2. Verfahren nach Anspruch 1, weiter umfassend:
(vi) Filtrieren der am Ende von Schritt (v) erhaltenen genannten homogenisierten Suspension durch ein Glasfaserfilter von 2 µ und Füllen des Filtrats in Glasfläschchen unter einer Stickstoffdecke, festes Verschließen der Fläschchen und Sterilisieren der fest verschlossenen Fläschchen mittels Autoklavieren zum Erhalt des Endproduktes, das zur parenteralen Verabreichung geeignet ist.

3. Verfahren nach Anspruch 1 oder 2, worin die Phospholipide aus Eiphosphatidylcholin (EPC) oder einem Gemisch aus Dimyristoylphosphatidylcholin (DMPC) und dem Natriumsalz des Dimyristoylphosphatidylglycerols (DMPG) ausgewählt sind.

4. Verfahren nach einem der vorangehenden Ansprüche, worin das genannte organische Lösungsmittel aus alkoholischen Lösungsmitteln, chlorierten Kohlenwasserstoffen und Gemischen davon ausgewählt ist.

5. Verfahren nach Anspruch 4, worin genanntes organisches Lösungsmittel aus Methanol, Ethanol, Isopropylalkohol, Chloroform, Tetrachlorkohlenstoff und Methylenchlorid ausgewählt ist.

6. Verfahren nach Anspruch 4, worin genanntes organisches Lösungsmittel Ethanol ist.

7. Verfahren nach einem der vorangehenden Ansprüche, worin der Amphotericin B-Gehalt von ca. 0,1 % bis 1 % (w/v) der Zusammensetzung beträgt.

8. Verfahren nach Anspruch 7, worin der Amphotericin B-Gehalt 0,5 % (w/v) der Zusammensetzung beträgt.

9. Verfahren nach einem der vorangehenden Ansprüche, worin der Natriumchlorid-Gehalt zwischen 0,1 % bis 0,9 % (w/v) der Zusammensetzung beträgt.

10. Verfahren nach Anspruch 9, worin der Natriumchloridgehalt zwischen 0,4 % bis 0,9 % (w/v) bezogen auf die Zusammensetzung beträgt.

11. Verfahren nach einem der vorangehenden Ansprüche, worin der Gehalt an Phospholipiden von ca. 0,1 % bis 1 % (w/v) der Zusammensetzung beträgt.

12. Verfahren nach einem der vorangehenden Ansprüche, worin der Gehalt an Phospholipiden 0,4 % bis 0,6 % (w/v) der Zusammensetzung beträgt.

13. Verfahren nach einem der vorangehenden Ansprüche, worin das Gewichtsverhältnis von Amphotericin B zu Phospholipiden von ca. 1 : 0,8 bis ca. 1 : 1,2 beträgt.

14. Verfahren nach einem der vorangehenden Ansprüche, worin Phospholipide Dimyristoylphosphatidylcholin (DMPC) und Dimyristoylphosphatidylglycerol (DMPG) in einem Gewichtsverhältnis von DMPC : DMPG von ca. 7 : 1 bis ca. 7 : 15 darstellen.

15. Verfahren nach Anspruch 14, worin das Gewichtsverhältnis von DMPC : DMPG 7 : 3 beträgt.

16. Verfahren nach einem der vorangehenden Ansprüche, worin nicht mikronisiertes Amphotericin B verwendet wird und die in Schritt (ii) nach Anspruch 1 verwendete parenteral verträgliche wässrige Phase Wasser oder Phosphatpuffer darstellt.

17. Verfahren nach einem der Ansprüche 1 bis 15, worin mikronisiertes Amphotericin B verwendet wird und die in Schritt (ii) nach Anspruch 1 verwendete parenteral verträgliche wässrige Phase Wasser, Phosphatpuffer, Kochsalzlösung oder phosphatgepufferte Kochsalzlösung darstellt.

18. Verfahren nach einem der vorangehenden Ansprüche, worin der pH der zur Suspension von Amphotericin B in Schritt (ii) verwendeten genannten wässrigen Phase auf 6,0 - 8,0 eingestellt ist.

19. Verfahren nach einem der vorangehenden Ansprüche, worin Sterilisation der homogenisierten filtrierten Suspension mittels konventionellen Autoklavierens durchgeführt wird.

20. Verfahren nach einem der vorangehenden Ansprüche, worin die Sterilisationstemperatur 110 °C beträgt.

21. Verfahren nach einem der Ansprüche 1 bis 18 und 20, worin die Sterilisation mittels eines Spezialautoklavierverfahrens durchgeführt wird, worin die Erhitzungs- und Abkühlungszeit mittels eines Schnellerhitzungs- und Schnellabkühlungszyklus reduziert wird.

22. Verfahren nach einem der Ansprüche 1 bis 15 und 17 bis 21, worin das Amphotericin B mikronisiert wird und Natriumchlorid in einem der Schritte (ii) bis (iv) zugefügt wird, damit der Natriumchlorid-Gehalt des Endproduktes mindestens 0,1 % (w/v) beträgt.

23. Verfahren nach einem der vorangehenden Ansprüche, worin das Produkt der wässrigen Zusammensetzung vollkommen frei von jeglichem chlorierten Kohlenwasserstoff ist.

24. Parenterale, Dimethylsulfoxid-freie wässrige Zusammensetzung von geringer Toxizität, enthaltend Amphotericin B, Natriumchlorid und Phospholipide, die mittels eines Verfahrens nach einem der vorangehenden Ansprüche erhältlich ist.

## Revendications

1. Procédé de fabrication d'une composition aqueuse parentérale dépourvue de sulfoxyde de diméthyle à faible toxicité contenant de l'amphotéricine B, du chlorure de sodium et des phospholipides, comprenant les étapes de :
(i) dissoudre un ou plusieurs phospholipides dans un ou plusieurs des solvants organiques acceptables de manière parentérale, puis éliminer les solvants par évaporation sous une pression réduite pour former un film sec des phospholipides uniques ou mélangés ;
(ii) suspendre l'amphotéricine B dans une phase aqueuse acceptable de manière parentérale, ne contenant pas de chlorure de sodium, ou suspendre l'amphotéricine B micronisée dans une phase aqueuse acceptable de manière parentérale, pouvant contenir du chlorure de sodium ;
(iii) ajouter la phase aqueuse contenant de l'amphotéricine B suspendue formée à la fin de l'étape (ii) audit film de phospholipides obtenu à la fin de l'étape (i) et mélanger les deux pour obtenir une suspension de ladite amphotéricine B avec lesdits phospholipides dans ladite phase aqueuse ;
(iv) ajuster le pH de ladite suspension obtenue à la fin de l'étape (iii) à de 6,0 à 8,0 puis l'homogénéiser jusqu'à ce qu'il devienne filtrable à travers un filtre en fibre de verre de 2 µ ; et
(v) ajouter suffisamment de solution de chlorure de sodium dans l'eau à la fin de l'étape (iv) de manière à ce que la teneur en chlorure de sodium du produit final soit au moins de 0,1 % p / v.

2. Procédé selon la revendication 1, comprenant en outre :
(vi) filtrer ladite suspension homogénéisée obtenue à la fin de l'étape (v) à travers un filtre en fibre de verre de 2 µ et verser le filtrat dans des flacons sous une couverture d'azote, sceller les flacons et stériliser les flacons scellés par passage à l'autoclave pour obtenir le produit final approprié pour une administration parentérale.

3. Procédé selon la revendication 1 ou la revendication 2, dans lequel les phospholipides sont choisis parmi la phosphatidylcholine d'oeuf (EPC) ou un mélange de phosphatidylcholine de dimyristoyl (DMPC) et de sel de sodium de dimyristoylphosphatidylglycérol (DMPG).

4. Procédé selon l'une quelconque des revendications précédentes, dans lequel ledit solvant organique est sélectionné parmi les solvants alcooliques, les hydrocarbures chlorés et les mélanges de ceux-ci.

5. Procédé selon la revendication 4, dans lequel ledit solvant organique est sélectionné parmi le méthanol, l'éthanol, l'alcool d'isopropyle, le chloroforme, le tétrachlorure de carbone et le chlorure de méthylène.

6. Procédé selon la revendication 4, dans lequel ledit solvant organique est de l'éthanol.

7. Procédé selon l'une quelconque des revendications précédentes, dans lequel la teneur en amphotéricine B varie d'environ 0,1 % à 1 % p / v de la composition.

8. Procédé selon la revendication 7, dans lequel la teneur en amphotéricine B est de 0,5 % p / v de la composition.

9. Procédé selon l'une quelconque des revendications précédentes, dans lequel la teneur en chlorure de sodium est entre 0,1 % à 0,9 % p / v de la composition.

10. Procédé selon la revendication 9, dans lequel la teneur en chlorure de sodium est entre 0,4 % à 0,9 % p / v de la composition.

11. Procédé selon l'une quelconque des revendications précédentes, dans lequel la teneur en phospholipides varie d'environ 0,1 % à 1 % p / v de la composition.

12. Procédé selon l'une quelconque des revendications précédentes, dans lequel la teneur en phospholipides est de 0,4 % à 0,6 % p / v de la composition.

13. Procédé selon l'une quelconque des revendications précédentes, dans lequel le rapport en poids d'amphotéricine B aux phospholipides varie d'environ 1 / 0,8 à environ 1 / 1,2.

14. Procédé selon l'une quelconque des revendications précédentes, dans lequel les phospholipides sont de la dimyristoylphosphatidylcholine (DMPC) et du dimyristoylphosphatidylglycérol (DMPG) en un rapport en poids DMPC / DMPG allant d'environ 7 / 1 à environ 7 / 15.

15. Procédé selon la revendication 14, dans lequel le rapport en poids DMPC / DMPG est de 7 / 3.

16. Procédé selon l'une quelconque des revendications précédentes, dans lequel l'amphotéricine B non micronisée est utilisée et la phase aqueuse acceptable de manière parentérale utilisée dans l'étape (ii) de la revendication 1 est de l'eau ou un tampon de phosphate.

17. Procédé selon l'une quelconque des revendications 1 à 15, dans lequel l'amphotéricine B micronisée est utilisée et la phase aqueuse acceptable de manière parentérale dans l'étape (ii) de la revendication 1 est de l'eau, un tampon de phosphate, une solution saline ou une solution saline de tampon de phosphate.

18. Procédé selon l'une quelconque des revendications précédentes, dans lequel le pH de ladite phase aqueuse utilisée pour la suspension de l'amphotéricine B à l'étape (ii) est ajusté à de 6,0 à 8,0.

19. Procédé selon l'une quelconque des revendications précédentes, dans lequel la stérilisation de la suspension filtrée homogénéisée est effectuée par passage en autoclave classique.

20. Procédé selon l'une quelconque des revendications précédentes, dans lequel la température de stérilisation est de 110 °C.

21. Procédé selon l'une quelconque des revendications 1 à 18 et 20, dans lequel la stérilisation est effectuée par un procédé de passage en autoclave spécialisé dans lequel la durée de chauffage et de refroidissement est réduite par un cycle de chauffage rapide et de refroidissement rapide.

22. Procédé selon l'une quelconque des revendications 1 à 15 et 17 à 21, dans lequel l'amphotéricine B est micronisée et le chlorure de sodium est ajouté dans l'une quelconque des étapes (ii) à (iv) de manière à ce que la teneur en chlorure de sodium du produit final soit au moins de 0,1 % p / v.

23. Procédé selon l'une quelconque des revendications précédentes, dans lequel le produit de composition aqueuse est totalement dépourvu de tout hydrocarbure chloré.

24. Composition aqueuse parentérale dépourvue de sulfoxyde de diméthyle à faible toxicité contenant de l'amphotéricine B, du chlorure de sodium et des phospholipides pouvant être obtenue par un procédé selon l'une quelconque des revendications précédentes.
